# EUROPEAN PATENT APPLICATION

(11) **EP 3 919 099 A1**
(43) Date of publication of application: **08.12.2021**
(21) Application number: 20747985.8
(22) Date of filing: 29.01.2020
(51) Int. Cl.: A61M 5/158

(54) **CANNULA INSERTER**

(30) Priority: 30.01.2019 JP 2019014567
(71) Applicant: Asahi Polyslider Company, Limited, Osaka 530-0005 (JP)
(72) Inventor: SEKI, Kazuharu, Tokyo 108-0074 (JP); SHIRAIWA, Shogo, Maniwa-shi, Okayama 719-3226 (JP); IMORI, Hirokazu, Maniwa-shi, Okayama 719-3226 (JP)
(74) Representative: Murgitroyd & Company
(86) International application number: PCT/JP2020/003210
(87) International publication number: WO 2020/158814

(57) **Abstract**

According to an embodiment of the present invention, there is provided a cannula inserter, comprising a cannula, a needle, a first movable body, a second movable body, and a biasing means comprising a first coil spring and a second coil spring in a casing, the needle being able to be moved in the cannula, wherein the first coil spring and the second coil spring each of which is in a compression state before a use are arranged side by side in parallel, the first coil spring is capable of extending in an advance direction of the needle to bias the first movable body, and the second coil spring is capable of extending in a retraction direction of the needle to bias the second movable body, wherein a non-piercing end of the needle is secured to the second movable body, and a non-piercing end of the cannula is configured to be pressed by at least one of the first movable body and the second movable body, wherein the compression state of the first coil spring is released such that the first movable body is biased by the first coil spring, thereby enabling the first movable body and the second movable body which are combined with each other to be moved in the advance direction of the needle, wherein, after a completion of a movement of the first movable body and the second movable body to a predetermined position in the advance direction, a combination of the first movable body with the second movable body is

## Description

### TECHNICAL FIELD

The disclosure relates to a cannula inserter. Specifically, the disclosure relates to a cannula inserter for administering a fluid such as a medicinal solution to a patient.

### BACKGROUND OF THE INVENTION

In recent years, it is said that the number of diabetic patients in the world will more continue to increase in a future. Specifically, the number of the diabetic patients in the world is 400 million or more people at present, and it is said that it will increase to about 700 million peoples after about 30 years. Since it is necessary to maintain a homeostasis of a blood glucose level for a diabetic treatment, an insulin administration is essential for the homeostasis maintenance.

As one of examples on a method for the insulin administration, a method in which an insulin pump, a cannula inserter, and the like are combined has been known. This method makes it possible to regularly administer a small amount of insulin through the cannula inserter using the insulin pump.

### PATENT DOCUMENTS (RELATED ART PATENT DOCUMENTS)

PATENT DOCUMENT 1: Japanese Unexamined Patent Application Publication No. 2014-508607

### DISCLOSURE OF THE INVENTION

### PROBLEMS TO BE SOLVED BY THE INVENTION

The inventors of the present application have recognized that a conventional cannula inserter used for regularly administering a small amount of insulin has a relatively complicated structure and thus it is difficult to assemble components of the conventional cannula inserter. A difficulty in assembling the components may result in a decrease in a production efficiency of the cannula inserter. Thus, it is desired to provide a cannula inserter having a simple structure.

Under these circumstances, an object of the present invention is to provide a cannula inserter having a simple structure.

### MEANS FOR SOLVING THE PROBLEMS

In order to achieve the above object, an embodiment of the present invention provides a cannula inserter, comprising
a cannula, a needle, a first movable body, a second movable body, and a biasing means comprising a first coil spring and a second coil spring in a casing, the needle being able to be moved in the cannula,
wherein the first coil spring and the second coil spring each of which is in a compression state before a use are arranged side by side in parallel, the first coil spring is capable of extending in an advance direction of the needle to bias the first movable body, and the second coil spring is capable of extending in a retraction direction of the needle to bias the second movable body,
wherein a non-piercing end of the needle is secured to the second movable body, and a non-piercing end of the cannula is configured to be pressed by at least one of the first movable body and the second movable body,
wherein the compression state of the first coil spring is released such that the first movable body is biased by the first coil spring, thereby enabling the first movable body and the second movable body which are combined with each other to be moved in the advance direction of the needle,
wherein, after a completion of a movement of the first movable body and the second movable body to a predetermined position in the advance direction, a combination of the first movable body with the second movable body is released such that the compression state of the second coil spring is configured to be able to be released, and
wherein the second movable body is biased by the second coil spring upon a release of its compression state, thereby enabling the second movable body to be moved in the retraction direction of the needle.

### BRIEF DESCRIPTION OF THE DRAWINGS

Fig. 1A is a perspective view schematically showing a cannula inserter according to an embodiment of the present invention, the cannula inserter being in a pre-motion state corresponding to a standby state of a needle and a cannula respectively.
Fig. 1B is a side view schematically showing a cannula inserter according to an embodiment of the present invention, the cannula inserter being in a pre-motion state corresponding to a standby state of a needle and a cannula respectively.
Fig. 1C is a partially exploded side view schematically showing a cannula inserter according to an embodiment of the present invention, the cannula inserter being in a pre-motion state corresponding to a standby state of a needle and a cannula respectively.
Fig. 1D is a schematical perspective view of a cannula inserter in which a casing cover member is not shown according to an embodiment of the present invention, the cannula inserter being in a pre-motion state corresponding to a standby state of a needle and a cannula respectively.
Fig. 1E is a perspective view schematically showing a first casing cover member as a component of a cannula inserter according to an embodiment of the present invention.
Fig. 1F is a perspective view schematically showing a second casing cover member as a component of a cannula inserter according to an embodiment of the present invention.
Fig. 1G is a perspective view schematically showing a first movable body as a component of a cannula inserter according to an embodiment of the present invention.
Fig. 1H is a perspective view schematically showing a second movable body as a component of a cannula inserter according to an embodiment of the present invention.
Fig. 1I is a perspective view schematically showing an engaging portion of a second movable body as a component of a cannula inserter according to an embodiment of the present invention, the engaging portion being configured to be able to be in an engagement with a protrusion of a first movable body.
Fig. 1J is a perspective view schematically showing a holding member of a second movable body as a component of a cannula inserter according to an embodiment of the present invention, the holding member being configured to be able to hold a cannula.
Fig. 1K is an exploded top plan view schematically showing a cannula inserter according to an embodiment of the present invention, the cannula inserter comprising a holding member configured to be able to hold a cannula.
Fig. 2A is a perspective view schematically showing a cannula inserter according to an embodiment of the present invention, the cannula inserter being in a first motion state corresponding to an advance state of a needle and a cannula respectively.
Fig. 2B is a partially exploded side view schematically showing a cannula inserter according to an embodiment of the present invention, the cannula inserter being in a first motion state corresponding to an advance state of a needle and a cannula respectively.
Fig. 2C is a schematical perspective view of a cannula inserter in which a casing cover member is not shown according to an embodiment of the present invention, the cannula inserter being in a first motion state corresponding to an advance state of a needle and a cannula respectively.
Fig. 2D is a schematical perspective view of a cannula inserter in which a casing cover member is not shown according to an embodiment of the present invention, the cannula inserter being in a first motion state corresponding to an advance state of a needle and a cannula respectively.
Fig. 3A is a perspective view schematically showing a cannula inserter according to an embodiment of the present invention, the cannula inserter being in a second motion state corresponding to a retraction state of a needle and an advance state of a cannula.
Fig. 3B is a side view schematically showing a cannula inserter according to an embodiment of the present invention, the cannula inserter being in a second motion state corresponding to a retraction state of a needle and an advance state of a cannula.
Fig. 3C is a partially exploded side view schematically showing a cannula inserter according to an embodiment of the present invention, the cannula inserter being in a second motion state corresponding to a retraction state of a needle and an advance state of a cannula.
Fig. 3D is a schematical perspective view of a cannula inserter in which a casing cover member is not shown according to an embodiment of the present invention, the cannula inserter being in a second motion state corresponding to a retraction state of a needle and an advance state of a cannula.
Fig. 3E is a schematical perspective view of a cannula inserter in which a casing cover member is not shown according to an embodiment of the present invention, the cannula inserter being in a second motion state corresponding to a retraction state of a needle and an advance state of a cannula.

### MODES FOR CARRYING OUT THE INVENTION

The inventors of the present application have diligently studied a cannula inserter having a simple structure. As a result, they have created a cannula inserter according to an embodiment of the present invention.

For a provision of a cannula inserter having a simple structure, the present invention has such a technical idea that "a movement of each of a cannula and a needle is controlled using two movable bodies which are respectively configured to be able to be biased by two coil springs which can extend in opposite directions (i.e., advance and retraction directions of the needle) to each other". Namely, the present invention has such a technical idea that "each movement of the cannula and the needle is controlled by a movement control of respective two coil springs which can extend in the opposite directions to each other, the two coil springs respectively serving to be able to move the two movable bodies".

The phrase "cannula inserter" as used herein means a device for inserting a cannula into a skin surface of a patient or the like for example. The phrase "cannula" as used herein means a flexible tube used for administering or injecting a medicinal solution such as insulin. The phrase "needle" as used herein means a component used to pierce the skin surface of the patient or the like. The phrase "movable body" as used herein means a structure which is capable of sliding by an external force (i.e., a biasing force of a coil spring), and can also be referred to as a sliding body. The phrase "advance direction of a needle" as used herein means a piercing direction of the needle. The phrase "retraction direction of a needle" as used herein means a direction opposite to the piercing direction of the needle. The phrase "coil spring" as used herein means a spring in a shape of a coil in a broad sense, and a compression coil spring serving to utilize a counterforce arising upon a release of its compression state in a narrow sense. Furthermore, each component of the cannula inserter according to an embodiment of the present invention may be composed of at least one of a resin component and a metal component.

### Configuration of Cannula Inserter

A main configuration of a cannula inserter 100 according to an embodiment of the present invention in accordance with the above technical idea will be described hereinafter with reference to drawings each showing a pre-motion state.

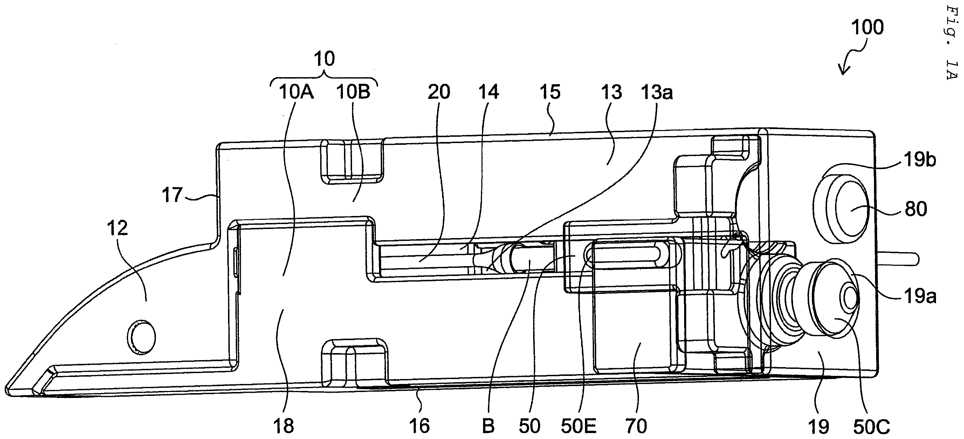

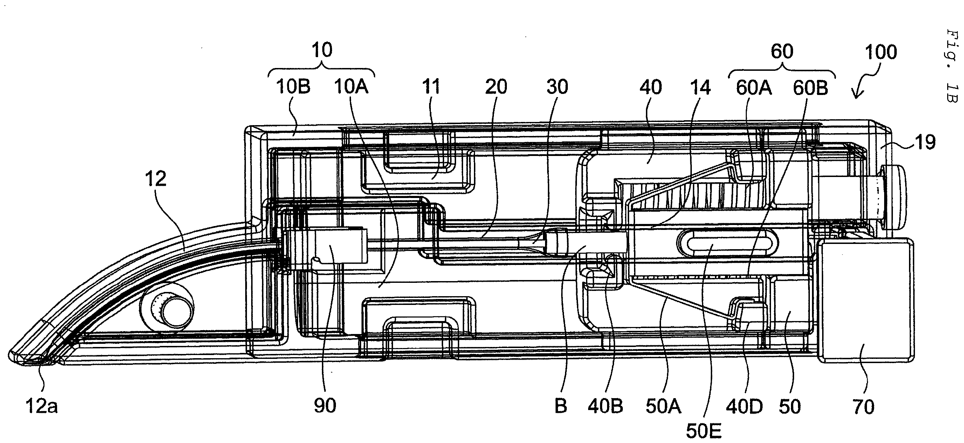

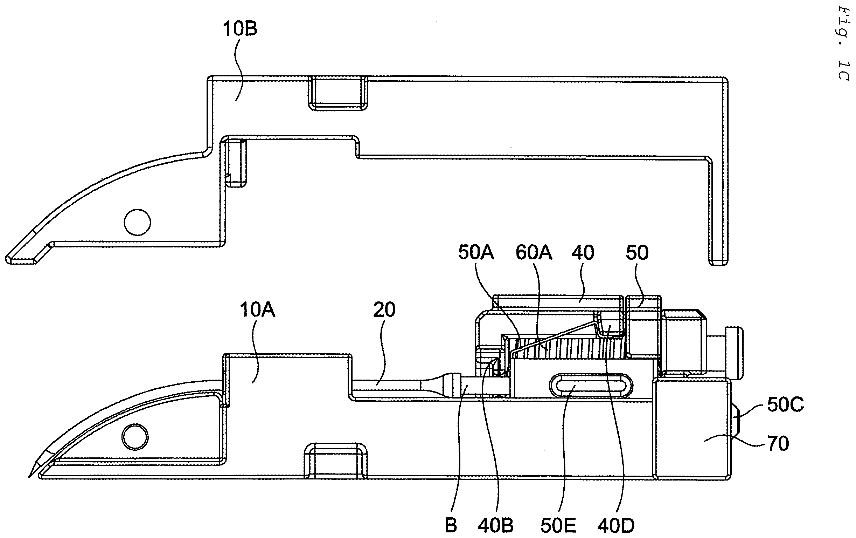

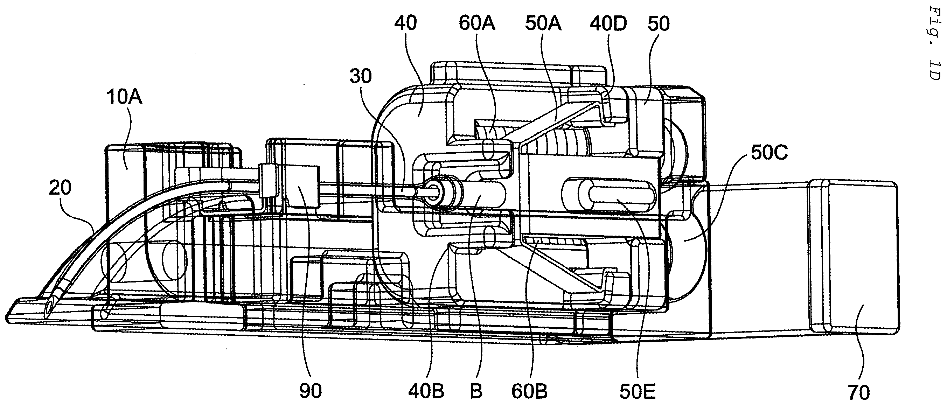

Fig. 1A is a perspective view schematically showing a cannula inserter according to an embodiment of the present invention, the cannula inserter being in a pre-motion state corresponding to a standby state of a needle and a cannula respectively. Fig. 1B is a side view schematically showing a cannula inserter according to an embodiment of the present invention, the cannula inserter being in a pre-motion state corresponding to a standby state of a needle and a cannula respectively. Fig. 1C is a partially exploded side view schematically showing a cannula inserter according to an embodiment of the present invention, the cannula inserter being in a pre-motion state corresponding to a standby state of a needle and a cannula respectively. Fig. 1D is a schematical perspective view of a cannula inserter in which a casing cover member is not shown according to an embodiment of the present invention, the cannula inserter being in a pre-motion state corresponding to a standby state of a needle and a cannula respectively.

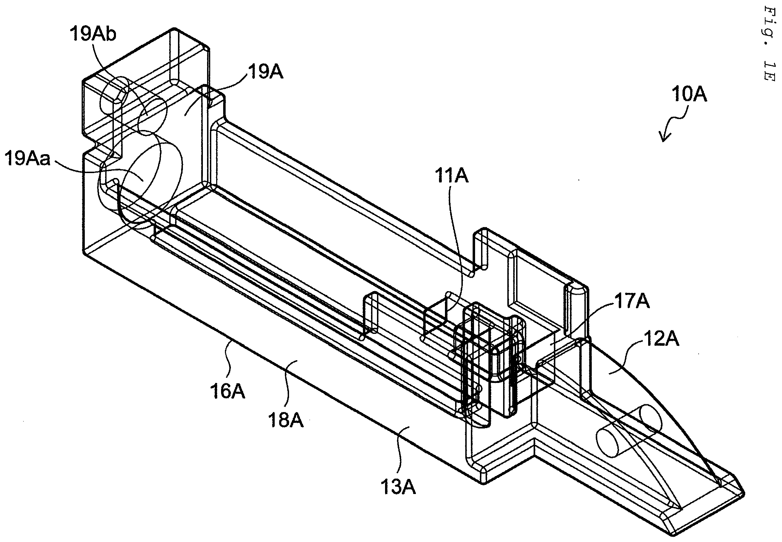

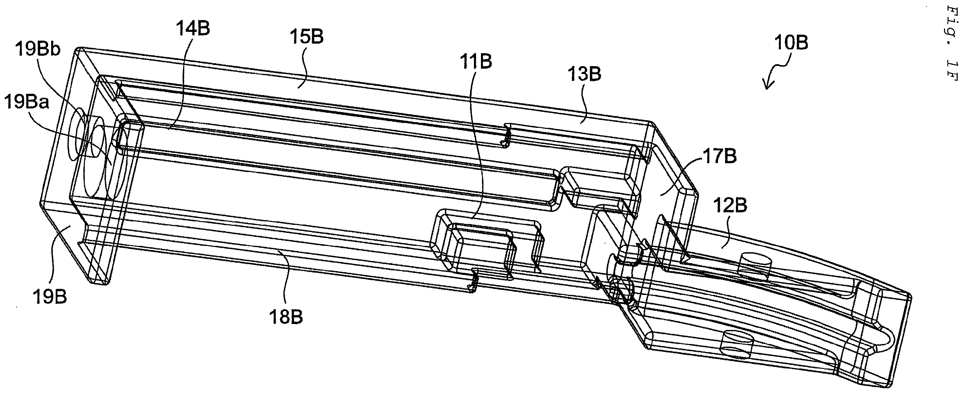

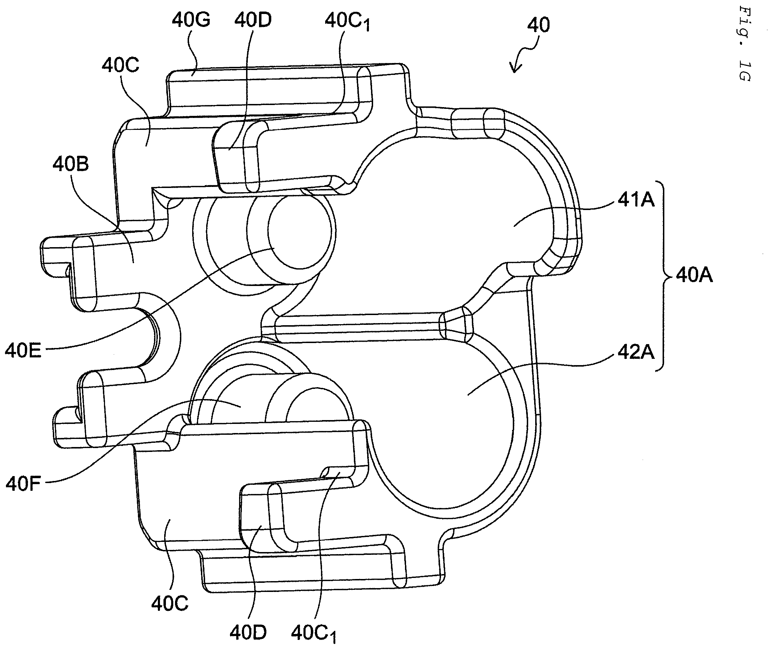

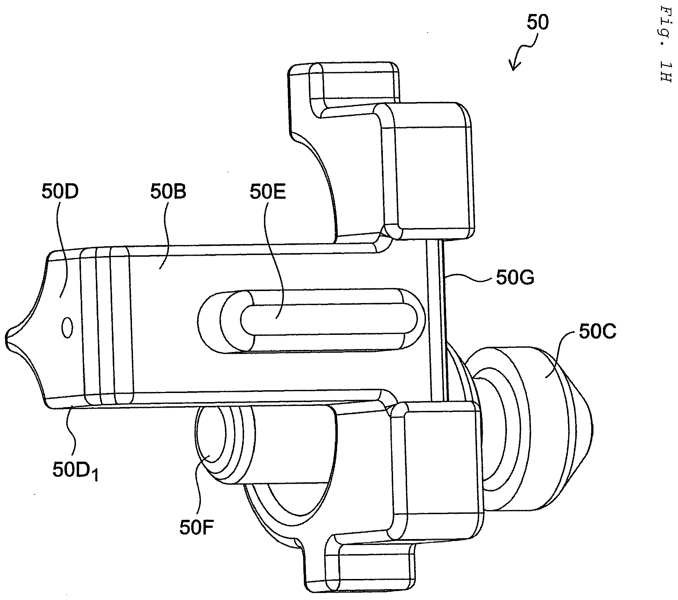

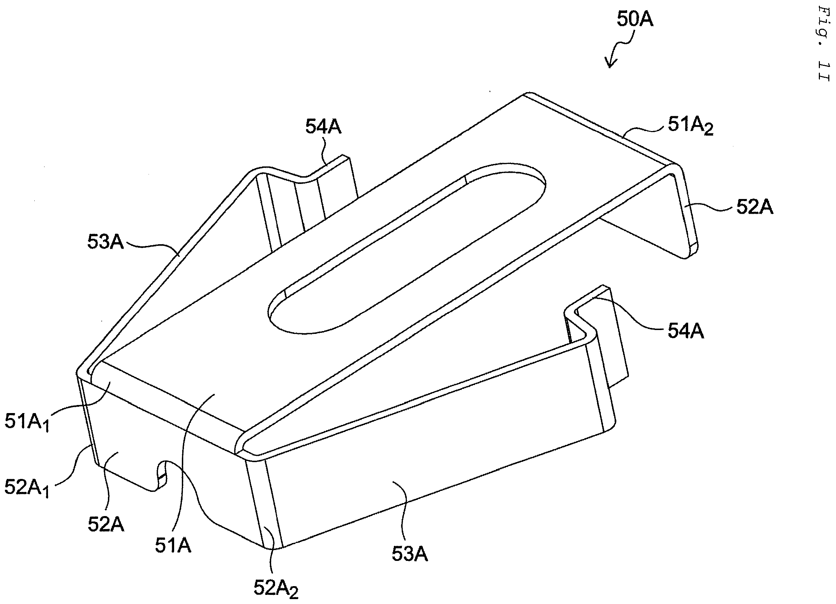

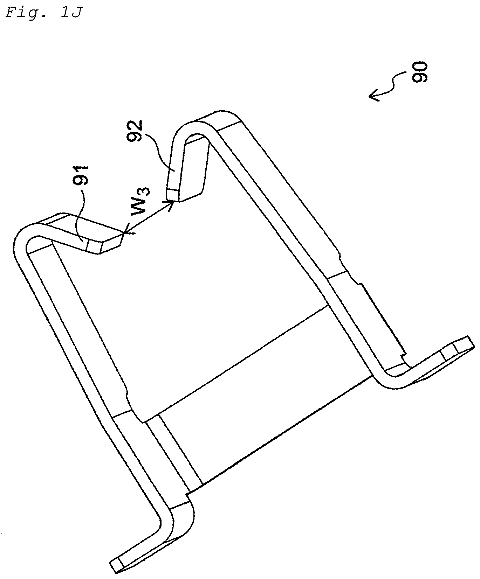

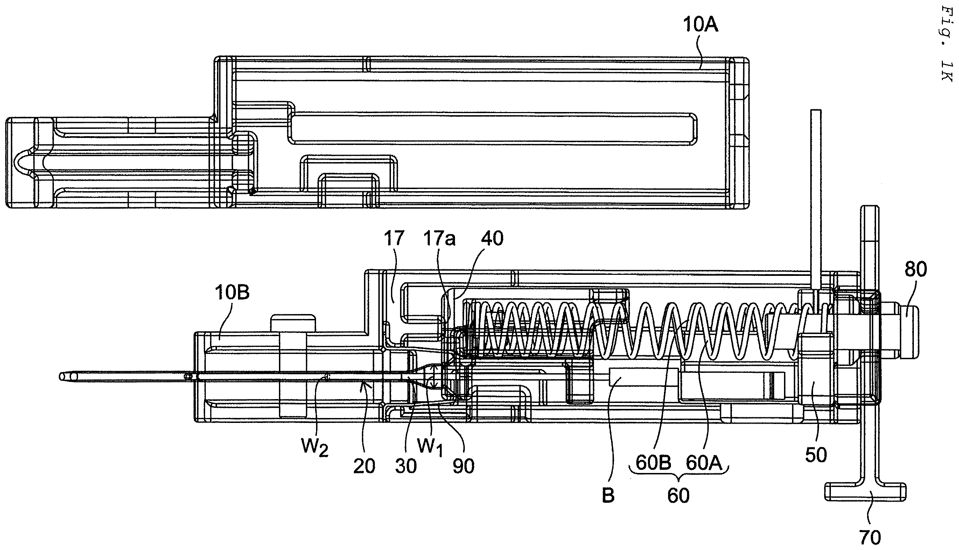

Fig. 1E is a perspective view schematically showing a first casing cover member as a component of a cannula inserter according to an embodiment of the present invention. Fig. 1F is a perspective view schematically showing a second casing cover member as a component of a cannula inserter according to an embodiment of the present invention. Fig. 1G is a perspective view schematically showing a first movable body as a component of a cannula inserter according to an embodiment of the present invention. Fig. 1H is a perspective view schematically showing a second movable body as a component of a cannula inserter according to an embodiment of the present invention. Fig. 1I is a perspective view schematically showing an engaging portion of a second movable body as a component of a cannula inserter according to an embodiment of the present invention, the engaging portion being configured to be able to be in an engagement with a protrusion of a first movable body. Fig. 1J is a perspective view schematically showing a holding member of a second movable body as a component of a cannula inserter according to an embodiment of the present invention, the holding member being configured to be able to hold a cannula. Fig. 1K is an exploded top plan view schematically showing a cannula inserter according to an embodiment of the present invention, the cannula inserter comprising a holding member configured to be able to hold a cannula.

As described in detail below, the cannula inserter 100 according to an embodiment of the present invention is one of components of a medicinal solution administration or dosing device. An example of the medicinal solution administration device is an insulin administration device. The insulin administration device may include an insulin pump and the like in addition to the cannula inserter 100.

The cannula inserter 100 according to an embodiment of the present invention includes a casing 10, and a cannula 20, a needle 30, a first movable body 40, a second movable body 50 and a biasing means 60 including a first coil spring 60A and a second coil spring 60B each provided in the casing 10 as main components of the cannula inserter 100, the needle 30 being able to be moved in the cannula 20 (see Figs. 1A to 1D). As will be described hereinafter, the cannula inserter 100 according to an embodiment of the present invention may further include a trigger member 70 and a holding member 90 as its components.

The second movable body 50 includes an engaging portion 50A having a flexibility as a component of the second movable body 50. As an example, the engaging portion 50A of the second movable body 50 may be a flexible wing portion. The phrase "wing portion" as used herein means a structure in a shape of a wing or a blade, the structure extending from side surfaces of a principal portion which corresponds to a main portion of the second movable body as described below.

The first movable body 40 includes a protrusion portion 40D as an engaged portion. According to this configuration, the engaging portion 50A of the second movable body can be engaged with the protrusion portion 40D of the first movable body 40, which enables the first movable body 40 and the second movable body 50 to be engaged with each other. Furthermore, the casing 10 includes a convex portion 11 on an inner surface of the casing 10. The convex portion 11 of the casing 10 is positioned to be able to be in a contact with the engaging portion 50A having the flexibility of the second movable body upon a completion of a movement thereof in an advance direction of the needle 30. The needle 30 is secured to the second movable body 50. The cannula 20 is configured to be pressed by at least one of the first movable body 40 and the second movable body 50.

According to an embodiment of the present invention, each of the first coil spring 60A and the second coil spring 60B of the biasing means 60 is in a compression state before a use thereof, and both coil springs in the compression state are arranged side by side in parallel. The first coil spring 60A in the compression state is capable of extending in the advance direction of the needle 30 to bias the first movable body 40. The second coil spring 60B in the compression state is capable of extending in a retraction direction of the needle 30 to bias the second movable body 50.

The first coil spring 60A is arranged between the casing 10 and the first movable body 40 such that one of end portions of the first coil spring 60A is secured to the casing 10 and other of the end portions of the first coil spring 60A is secured to the first movable body 40. According to the above arrangement, the first coil spring 60A in the compression state can extend from the one end side thereof secured to the casing 10 toward the other end side thereof secured to the first movable body 40 upon a release of its compression state.

The second coil spring 60B is arranged between the first movable body 40 and the second movable body 50 such that one of end portions of the second coil spring 60B is secured to the first movable body 40 and other of the end portions of the second coil spring 60B is secured to the second movable body 50. According to the above arrangement, the second coil spring 60B in the compression state can extend from the one end side thereof secured to the first movable body 40 toward the other end side thereof secured to the second movable body 50 upon a release of its compression state.

According to an embodiment of the present invention, the compression state of the first coil spring 60A is released such that the first movable body 40 is biased by the first coil spring 60A, thereby enabling the first movable body 40 and the second movable body 50 which are combined with each other to be moved in the advance direction of the needle 30. As described above, the first movable body 40 and the second movable body 50 are configured to be mutually engaged to be able to be combined with each other.

According to an embodiment of the present invention, after a completion of a movement of the first movable body 40 and the second movable body 50 to a predetermined position in the advance direction, a combination of the first movable body 40 with the second movable body 50 is released such that the compression state of the second coil spring 60B is configured to be able to be released. The release of the combination may result from a release of the engagement of the first movable body 40 with the second movable body 50.

Namely, an embodiment of the present invention makes it possible to suitably control an action timing of the biasing force of the one of the coil springs (i.e., the first coil spring 60A) which can extend in the advance direction of the needle 30 acts and an action timing of the biasing force of the other of the coil springs (i.e., the second coil spring 60B) which can extend in the retraction direction of the needle 30.

The release of the engagement of the first movable body 40 and the second movable body 50 can be achieved by the following means. Specifically, as described above, the convex portion 11 on the inner surface of the casing 10 is configured to be able to be in the contact with the flexible engaging portion 50A of the second movable body 50 upon the completion of the movement in the advance direction. According to the configuraton, upon the completion of the advance movement of the second movable body 50, the engaging portion 50A of the second movable body 50 can be in the contact with the convex portion 11 of the casing 10, which enables the flexible engaging portion 50A to be flexed. Thus, each engaging portion 50A can be spaced apart from the protrusion portion 40D of the first movable body 40, and thus the engagement of the first movable body 40 with the second movable body 50 can be released. Thus, according to an embodiment of the present invention, the second movable body 50 can be biased by the second coil spring 60B in the compression-release state, which enables the second movable body 50 to be moved in the retraction direction of the needle 30.

According to the above configuration, the following effects can be achieved.

### (Biasing force-action of first coil spring 60A => Launch of needle and cannula)

As described above, according to an embodiment of the present invention, the first movable body 40 can be biased by the first coil spring 60A which is capable of extending in the advance direction of the needle 30. The needle 30 is secured to the second movable body 50, and the cannula 20 is configured to be pressed by at least one of the first movable body 40 and the second movable body 50. Furthermore, the biasing of the first movable body 40 by the first coil spring 60A enables the first movable body 40 and the second movable body to be moved together in the advance direction of the needle 30.

In light of the above matters, when the compression state of the first coil spring 60A is released, the first coil spring 60A extends in the advance direction of the needle 30 and thereby the first movable body 40 is biased in the advance direction. Thus, the first movable body 40 can be moved in the advance direction. Due to the advance movement of the first movable body 40, the second movable body 50 combined with the first movable body 40 can be also moved in the advance direction of the needle 30.

Furthermore, since the needle 30 is secured to the second movable body 50, it is possible to move the needle 30 in the advance direction upon the advance movement of the second movable body 50. The cannula 20 is configured to be pressed by at least one of the first movable body 40 and the second movable body 50. Thus, it is possible to move the cannula 20 in the advance direction upon the advance movement of at least one of the first movable body 40 and the second movable body 50.

Namely, due to the movement of the first movable body 40 and the second movable body 50 which are combined with each other in the advance direction, both of the needle 30 and the cannula 20 can be moved in the advance direction. Accordingly, it is possible to launch or fire the cannula 20 and the needle 30 positioned in the cannula 20 to an outside (e.g., into the skin surface of the patient or the like).

### (Biasing force-action of second coil spring 60B => Retraction of needle)

As described above, according to an embodiment of the present invention, after the movement completion of the first movable body 40 and the second movable body 50 to the predetermined position in the advance direction, the combination of the first movable body 40 with the second movable body 50 is released and thereby the compression state of the second coil spring 60B can be released. The movement completion of the first movable body 40 results from a release completion of the compression state of the first coil spring 60A serving to bias the first movable body 40. Thus, the compression state of the second coil 60B spring 60B can be released after the release of the compression state of the first coil spring 60A.

The second coil spring 60B is configured to be able to extend in the retraction direction of the needle 30 to bias the second movable body 50. Thus, the second movable body 50 is biased by the second coil spring 60B upon the release of the compression state thereof. As a result, it is possible to move the second movable body 50 back in the retraction direction of the needle 30.

As described above, the needle 30 is secured to the second movable body 50, whereas the cannula 20 is configured to be pressed in the advance direction by at least one of the first movable body 40 and the second movable body 50. This means that the cannula 20 is pressed by the second movable body 50 whereas it is not secured to the second movable body 50.

According to such the configuration, upon the movement of the second movable body 50 in the retraction direction, it is possible to move only the needle 30 secured to the second movable body 50 in the retraction direction without a retraction movement of the cannula 20 whose advance movement has been made. Accordingly, it is possible to shift only the needle 30 from its launch state to an outside to its retraction state, while remaining to be a state where the cannula 20 is launched to the outside.

In light of the above matters, according to an embodiment of the present invention, in a state where a combination of the two movable bodies is obtained, due to the combination thereof, the both of the two movable bodies in the advance direction can be moved by the biasing means (i.e., the coil springs) . Thus, it is possible to launch both of the needle 30 and the cannula 20 to the outside of the casing 10 (e.g., the skin surface of the patient or the like) . While on the other hand, in a state where the combination of the two movable bodies is released, the release of the combination makes it possible to independently control a movement of only one of the movable bodies. Accordingly, it is possible to shift only the needle 30 from its launch state to the outside to its retraction state, while remaining to be the state where the cannula 20 is launched to the outside.

Particularly, according to an embodiment of the present invention, "the advance movement of the cannula 20 and the needle 30" and "the retraction movement of only the needle 30" results from a simple configuration of "the first movable body 40" and "the second movable body 50" which can be combined with each other, as well as each timing control for a keeping and a release of the combination thereof, "the first movable body 40" being biased by the first coil spring 60A, "the second movable body 50" being biased by the second coil spring 60B which can extend in the retraction direction of the needle 30. The simple configuration makes it possible to easily assemble the components with each other as a whole. Accordingly, it is possible to suitably improve a production efficiency of the cannula inserter according to an embodiment of the present invention.

### [Components of cannula inserter]

Components of the cannula inserter according to an embodiment of the present invention will be more specifically described hereinafter.

### (Casing 10)

As shown in Figs. 1A to 1F and Fig. 1K, the casing 10 includes a first casing cover member 10A and a second casing cover member 10B. The casing 10 includes a casing protrusion portion 12 formed at a front region of the casing 10. The casing 10 includes a casing main portion 13 other than the casing protrusion portion 12, the casing main portion 13 having an internal space region 13a which is capable of housing each component. The casing main portion 13 has an external configuration in a substantially rectangular parallelepiped shape. The internal space region 13a is capable of housing the cannula 20, the needle 30 which is movable in the cannula 20, the first movable body 40, the second movable body 50, and the biasing means 60. The internal space region 13a is formed by an engagement of the first casing cover member 10A with the second casing cover member 10B with each other for a combination of them.

The internal space region 13a is composed of an upper wall 15, a lower wall 16, a front wall 17, two side walls 18 faced to each other, and a rear wall 19 of the casing main portion 13. The front wall 17 includes a convex region 17a configured to be able to contact with the first movable body 40 which has been moved in the advance direction (see Fig. 1K) . The front wall 17 of the casing main portion 13 and the casing protruding portion 12 are continuous with each other. The casing main portion 13A partially has a clearance 14 extending in the advance or retraction direction of the needle 30 on a surface of the casing main portion 13A, which enables a convex portion 40G on a surface of the first movable body 40 and/or a convex portion 50E on a surface of the second movable body 50 to be moved in the clearance 14.

Namely, the clearance 14 on the surface of the casing 10 can function as a guide space region for guiding the movement of the convex portion 40G of the first movable body 40 and/or the convex portion 50E of the second movable body 50. The movement of the convex portion of each movable body in the clearance 14 makes it possible to suitably control a movement of each movable body in a predetermined moving direction. In other words, a steady sliding movement of each movable body can be made in the predetermined direction (i.e., advance/retraction direction).

The casing 10, specifically the casing main portion 13 of the casing 10 has the convex portion 11 on its inner surface. The convex portion 11 is positioned to be able to be in the contact with the engaging portion 50A having the flexibility of the second movable body 50 upon the completion of the movement thereof in the advance direction of the needle 30. As one of examples, convex portions 11 may be formed at a front region of the casing main portion 13 and also may be formed on an inner surface of the upper wall 15 and that of the lower wall 16 respectively.

The first casing cover member 10A composes a portion 12A of the casing protruding portion and a portion 13A of the casing main portion. The portion 13A of the casing main portion 13 includes a lower wall 16A, a front wall 17A, two side walls 18A opposed to each other, and a rear wall 19A. The front wall 17A and the portion 12A of the casing protruding portion are continuous with each other. The first casing cover member 10A includes a convex portion 11A on its inner surface. The convex portion 11A is positioned to be able to contact with the flexible engaging portion 50A of the second movable body 50 upon the completion of the movement in the advance direction of the needle 30. As an example, the convex portion 11A may be formed at a front region of the portion 13A of the casing main portion 13 and also may be formed on an inner surface of the lower wall 16A.

Similarly, the second casing cover member 10B composes a portion 12B of the casing protruding portion and a portion 13B of the casing main portion. The portion 13B of the casing main portion 13 includes an upper wall 15B, a front wall 17B, two side walls 18B opposed to each other, and a rear wall 19B. The front wall 17B and the portion 12B of the casing protruding portion are continuous with each other. The second casing cover member 10B includes a convex portion 11B on its inner surface. The convex portion 11B is positioned to be able to contact with the flexible engaging portion 50A of the second movable body 50 upon the completion of the movement in the advance direction of the needle 30. As an example, the convex portion 11B may be formed at a front region of the portion 13B of the casing main portion 13 and also may be formed on an inner surface of the upper wall 15B.

Due to the convex portions 11A, 11B, upon the completion of the advance movement of the second movable body 50, engaging portions 50A of the second movable body 50 can be in the contact with the convex portion 11A of the first casing cover member 10A and the convex portion 11B of the second casing cover member 10B, respectively, thereby enabling the flexible engaging portions 50A to be flexed. Thus, the engaging portions 50A can be spaced apart from the protrusion portions 40D of the first movable body 40, and thus the engagement of the first movable body 40 with the second movable body 50 can be released.

The portion 13B of the casing main portion partially has a clearance 14B extending in the advance direction of the needle 30 on a surface of the portion 13B, which enables a convex portion 40G of the first movable body 40 to be moved in the clearance 14B. Thus, it is possible to suitably control a movement of the first movable body 40 in the predetermined moving direction.

The rear wall 19 of the casing main portion 13, i.e., the rear wall 19 of the casing 10 has two openings 19a, 19b. The two openings 19a, 19b are composed of two sub-openings 19Aa, 19Ab in the rear wall 19A of the first casing cover member 10A and two sub-openings 19Ba, 19Bb in the rear wall 19B of the second casing cover member 10B which are in an alignment with each other.

The one opening 19a of the casing 10 is configured such that an extension portion 50C of the second movable body 50, which will be described later, can pass through the one opening 19a in back and forth directions. The other opening 19b serves to insert a portion of a shaft member 80 to which one end of the first coil spring 60A is secured into an internal space region of the casing 10. The shaft member 80 is secured to the casing 10 after being inserted. It should be noted that the shaft member 80 and the opening 19b are not essential, and the first coil spring 60A may be directly secured to the inner surface of the casing 10.

The casing protrusion portion 12 has an internal passage through which the needle 30 and the cannula 20 can pass, and has an opening 12a on a surface for launching the needle 30 and the cannula 20 to the outside (e.g., the skin surface of the patient or the like) . The casing protrusion portion 12 is composed of a protrusion portion 12A at a front region of the first casing cover member 10A and a protrusion portion 12B at a front region of the second casing cover member 10B.

The internal passage of the casing protrusion portion 12 through which the needle 30 and the cannula 20 can pass has an inwardly curved shape. The needle 30 and the cannula 20 whose at least piercing end side is in a shape of an inward curve are configured to be able to pass through the internal passage having the inwardly curved shape. While not being particularly limited, the casing protrusion portion 12 may have a surface of a curved shape corresponding to the inward curved shape of the internal passage.

Namely, according to an embodiment of the present invention, it is possible to use the needle 30 and the cannula 20 whose at least piercing end side is in the shape of the inward curve. Due to the inwardly curved shape, at least the respective piercing end sides of the needle 30 and the cannula 20 may extend in a direction different from respective axial directions of the first coil spring 60A moving in the advance direction and the second coil spring 60B moving in the retraction direction, not in a direction parallel to the respective axial directions.

Furthermore, due to the inwardly curved shape, at least the respective piercing end sides of the needle 30 and the cannula 20 are pierced in a direction oblique to the skin surface of the patient or the like, not in a direction perpendicular to the skin surface. If the needle 30 and the cannula 20 extend in a direction parallel to the respective axial directions of the first coil spring 60A moving in the advance direction and the second coil spring 60B moving in the retraction direction, thereby having a linear configuration as a whole, they are pierced in the direction perpendicular to the skin surface. In light of the above matters, according to an embodiment of the present invention, the piercing in the oblique direction makes an incidence angle of the cannula 20 and the needle 30 into the skin surface smaller. Thus, a prickle felt by the patient or the like can be reduced upon the piercing into the skin.

In the pre-motion state of the cannula inserter 100 according to an embodiment of the present invention as shown in Fig. 1B, it is preferable that each tip of the cannula 20 and the needle 30 is positioned in the casing protrusion portion 12 such that they are not accidentally pierced into the skin surface of the user or the like.

Furthermore, it is preferable that an opening of the insulin administration device having the cannula inserter 100 therein is sealed with a silicon film or the like in order to avoid an intrusion of a contaminant (e.g., contaminated liquid and/or contaminated gas, etc.) into the opening 12a before the launch of the cannula 20 and the needle 20. It is also preferable that a position of the opening of the insulin administration device and that of the opening 12a of the casing protrusion portion 12 are the same as each other in order to suitably launch the needle 30 and the cannula 20 to the outside (e.g., to the skin surface of the patient or the like).

### (First movable body 40)

As shown in Figs. 1A to 1D and Fig. 1G, the first movable body 40 is slidable in a direction (i.e., advance direction) in the casing 10 and can be combined with the second movable body 50. The first movable body 40 includes a support portion 40A, a front wall portion 40B, a side wall portion 40C, a protrusion portion 40D with which the engaging portion of the second movable body 50 can be engaged, and a convex portion 40G formed on a surface of the first movable body 40. The support portion 40A is configured to be able to support the biasing means 60. The front wall portion 40B extends in a substantially vertical direction from one end side of the support portion 40A. The side wall portion 40C is in a contact with the front wall portion 40B and extends substantially vertical direction from at least one of side regions of the support portion 40A. The protrusion portion 40D is formed at an end region 40C₁ of at least one of the side wall portions 40C, for example. The convex portion 40G protrudes from a surface of the side wall portion 40C, for example, and is configured to be able to be moved in the clearance 14 formed on the surface of the casing 10 (specifically, the surface of the casing main portion 13) in the advance direction or the retraction direction of the needle 30. The "end region 40C₁ of the side wall portion 40C" as used herein means an end region opposite to the end region in contact with the front wall portion 40B.

The support portion 40A of the first movable body 40 includes support regions 41A, 42A capable of supporting the biasing means 60 (i.e., the first coil spring 60A and second coil spring 60B). The front wall portion 40B of the first movable body 40 includes two protrusions 40E, 40F on an inner side surface of the front wall portion 40B. The one protrusion portion 40E is a protrusion to which one end-side of the first coil spring 60A can be secured or connected. The other protrusion portion 40F is a protrusion to which one end-side of the second coil spring 60B can be secured or connected.

As described above, the other end-side of the first coil spring 60A is secured to the inner surface side of the casing 10. Thus, the first coil spring 60A is arranged between the casing 10 and the first movable body 40 in the state of the securement of both ends of the first coil spring 60A. According to the arrangement of the first coil spring 60A, upon the release of the compression of the first coil spring 60A, the compressed first coil spring 60A is capable of extending from the one end side secured to the casing 10 toward the other end side secured to the first movable body 40. Furthermore, as described above, the other end-side of the second coil spring 60B is secured to the second movable body 50. Thus, the second coil spring 60B is arranged between the first movable body 40 and the second movable body 50 in the state of the securement of both ends of the second coil spring 60B. According to the arrangement of the second coil spring 60B, upon the release of the compression of the second coil spring 60B, the compressed second coil spring 60B is capable of extending from the one end side secured to the first movable body 40 toward the other end side secured to the second movable body 50.

### (Second movable body 50)

As shown in Figs. 1A to 1D, 1H and 1I, the second movable body 50 includes the engaging portion 50A having the flexibility which can be engaged with the protrusion portion 40D of the first movable body 40, a body portion 50B, an extension portion 50C which is movable to an outside of the casing 10 or to an inside of the casing 10, and a protrusion 50F formed on the body portion 50B.

As shown in Figs. 1H and 1I, the body portion 50B and the engaging portion 50A can be composed of separate members. Namely, the body portion 50B and the engaging portion 50A can be separated before the use, and then upon the use, both may be combined with each other as shown in Figs. 1A to 1D. The combination of the body portion 50B with the engaging portion 50A results from respective engagements of a front wall 50D and a rear wall 50G of the body portion 50B with two side surfaces 52A of the engaging portion 50A, the two side surfaces being opposed to each other.

According to the above configuration, it is not necessary to produce a unified product having a complicated structure at one time and thus each member can be produced separately, which enables a production efficiency to be improved. Without being not limited to the above, the body portion 50B and the engaging portion 50A may be inseparably unified regardless of before and after the use.

While being not particularly limited, the engaging portion 50A of the second movable body 50 may be a flexible wing portion. In this case, the wing portion may be configured to be able to be positioned between the front wall portion 40B and the protrusion portion 40D of the first movable body 40 for the combination of the first movable body 40 with the second movable body 50.

The engaging portion 50A such as the wing portion of the second movable body 50 includes a main surface 51A, respective side surfaces 52A extending in a direction substantially perpendicular to the main surface 51A from an front end 51A₁ and a rear end 51A₂ of the main surface 51A, a wing 53A extending from at least one of end portions 52A₁, 52A₂ of the side surface 52A. The wing 53A has a latch 54A at its end portion, and the latch 54A is configured to be able to be engaged with the protrusion portion 40D of the first movable body 40. The engagement of the latch 54A with the protrusion portion 40D enables the first movable body 40 with the second movable body 50 to be engaged with each other as a whole. Thus, the combination of the first movable body 40 with the second movable body 50 is possible as described above.

Furthermore, the one of two side surfaces 52A of the engaging portion 50A can be engaged with the front wall 50D of the body portion 50B of the second movable body 50, and the other of the two side surfaces 52A can be engaged with the rear wall 50G of the body portion 50B. Thus, it is possible to combine the body portion 50B of the second movable body 50 with the engaging portion 50A thereof. In the combined state, the wing 53A is configured to extend in a wing shape from at least one of end regions 50D₁ of the front wall 50D of the body portion 50B of the second movable body 50 as shown in Figs. 1B to 1D.

A non-piercing end of the needle 30 is secured to the second movable body 50. As one of examples, the non-piercing end of the needle 30 is secured to the body portion 50B of the second movable body 50. The non-punctured end of the needle 30 can be attached to the second movable body 50. For example, the non-punctured end of the needle 30 can be attached to the body portion 50B of the second movable body 50. The body portion 50B includes the front wall 50D and the rear wall 50G. The front wall 50D and the rear wall 50G are configured to be able to be engaged with the two side surfaces 52A opposed to each other of the engaging portion 50A of the second movable body 50 as described above.

The extension portion 50C of the second movable body 50 extends from a rear end side of the body portion 50B to the opening 19a to be able to pass therethrough in the back and forth directions, the opening 19a being formed in the rear wall 19 of the casing 10. The protrusion 50F is a protrusion to which the other end side of the second coil spring 60B can be secured. While a position of the protrusion 50F is not particularly limited as long as the other end side of the second coil spring 60B can be secured, the protrusion 50F may extend in a direction which is substantially the same axial direction as that of the extension portion 50C as shown in Fig. 1H, for example.

As described above, at least one of the first movable body 40 and the second movable body 50 is configured to be able to press the cannula 20. As an example, as shown in Figs. 1A to 1D, the second movable body 50 is configured to press the cannula 20 in the advance direction via an intercalated member. Specifically, as an example, the front wall portion 50D of the second movable body 50 is configured to be able to press the cannula 20 in the advance direction via the intercalated member B. The intercalated member B is not necessarily an indispensable member, and the front wall portion 50D of the second movable body 50 may be able to directly press the cannula 20 in the advance direction. Furthermore, according to an embodiment of the present invention, a configuration of the cannula 20 is not particularly limited if its advance movement is possible. While the drawing shows an embodiment wherein the second movable body 50 presses the cannula 20 in the advance direction via the intercalated member B, only the first movable body 40 or both of the first movable body 40 and the second movable body may be able to directly or indirectly press the cannula 20.

### (Trigger member 70)

The cannula inserter 100 according to an embodiment of the present invention may further include the trigger member 70 as one of the components of the cannula inserter 100.

In a case where the first movable body 40 and the second movable body 50 are combined with each other, the trigger member 70 may be configured to be able to hold at least one of the first movable body 40 and the second movable body 40 before the advance movement thereof in the pre-motion state. In other words, the at least one of the first movable body 40 and the second movable body 40 before the advance movement thereof may be held by the trigger member 70.

For example, the trigger member 70 may be configured such that the extension portion 50C of the second movable body 50 is capable of being inserted and subsequently held therein as shown in Figs. 1A to 1D. While an installation position of the trigger member 70 is not particularly limited, as an example, the trigger member 70 may be positioned to be able to hold the extension portion 50C of the second movable body 50 by an insertion of the trigger member 70 through a clearance formed at a rear end side of the side wall 18 of the casing main portion 13.

When the trigger member 70 holds the extension portion 50C of the second movable body 50 for example, a movement of the second movable body 50 can be stopped at the rear wall 19 of the casing 10. Furthermore, the combination of the second movable body 50 with the first movable body 40 together in the pre-motion state makes it possible to stop a movement of the first movable body 40 as well as the second movable body 50. Thus, it is possible to subject both of the first movable body 40 and the second movable body 50 combined together as a whole, to be in a stationary state at the rear wall 19 side of the casing 10.

An embodiment on the trigger member 70 is not particularly limited as long as the at least one of the first movable body 40 and the second movable body 50 combined with each other is held by the trigger member 70 in the pre-motion state. For example, the trigger member 70 may be configured to be able to hold the at least one of the first movable body 40 and the second movable body 50 combined with each other by an insertion of the trigger member 70 through the upper wall 15 or the lower wall 16 other than the side wall 18 of the casing main portion 13.

### (Biasing means 60)

As described above, the biasing means 60 includes the first coil spring 60A and the second coil spring 60B. Each of the first coil spring 60A and the second coil spring 60B is in the compression state before the use thereof, and both coil springs respectively in the compression state are arranged side by side in parallel. The first coil spring 60A and the second coil spring 60B arranged side by side in parallel may have the substantially same diameter as each other. The substantially same diameter as each other enables the second coil spring 60B not to be placed in a space of the first coil spring 60A.

The first coil spring 60A is arranged between the casing 10 and the first movable body 40 such that the one of the end portions of the first coil spring 60A is secured to the casing 10 and the other of the end portions of the first coil spring 60A is secured to the first movable body 40. The first coil spring 60A in the compression state is capable of extending in the advance direction of the needle 30 to bias the first movable body 40. According to the above arrangement, the first coil spring 60A in the compression state can extend from the one end side thereof secured to the casing 10 toward the other end side thereof secured to the first movable body 40 upon the release of its compression state. Thus, the first movable body 40 can be biased in the advance direction by the first coil spring 60A.

In particular, according to an embodiment, upon the compression release of the first coil spring 60A, the first movable body 40 and the second movable body 50 are combined with each other. As described above, according to an embodiment of the present invention, the at least one of the first movable body 40 and the second movable body 50 combined together with each other is held by the trigger member 70. As an example, the trigger member 70 is configured to be able to hold the extension portion 50C of the second movable body 50. When its holding state is released, a holding relationship between the second movable body 50 and the trigger member 70 is disengaged. This specically means a disengagement of a holding relationship between the trigger member 70 and the combined body of "the first movable body 40 combined together with the second movable body 50" and "the second movable body 50".

Since the first coil spring 60A can bias the first movable body 40 in the advance direction, it is possible to make the advance movement of the combined body of the first movable body 40 and the second movable body 50 combined with the first movable body 40. Namely, both of the first movable body 40 and the second movable body 40 can be moved in the advance direction. As described above, the non-piercing end of the needle 30 is secured to the second movable body 50 and the non-piercing end of cannula 20 is configured to be pressed by the at least one of the first movable body 40 and the second movable body 50. Thus, the advance movement of the first movable body 40 and the second movable body 50 both enables both of the needle 30 and the cannula 20 to be moved in the advance direction.

It is preferable that the first coil spring 60A has the biasing force which enables the first movable body 40 to be contacted with the inner surface of the front wall 17 of the casing main portion 13 in the advance direction of the needle 30. Thus, both of the cannula 20 and the needle 30 can be suitably launched from the casing 10, specifically the casing protrusion portion 12 to the outside of the casing 10.

The second coil spring 60B is arranged between the first movable body 40 and the second movable body 50 such that the one of the end portions of the second coil spring 60B is secured to the first movable body 40 and the other of the end portions thereof is secured to the second movable body 50. The second coil spring 60B in the compression state is capable of extending in the retraction direction of the needle 30 to bias the second movable body 50. According to the above arrangement, the second coil spring 60B in the compression state can extend from the one end side thereof secured to the first movable body 40 toward the other end side thereof secured to the second movable body 50 upon the release of its compression state. Thus, the second movable body 50 can be biased in the retraction direction by the second coil spring 60B.

In particular, according to an embodiment, upon the compression release of the second coil spring 60B, the combination of the both movable bodies have been released by the advance movement-completion of each of the first movable body 40 and the second movable body 50. Thus, upon the compression release of the second coil spring 60B, the biasing of the second coil spring 60B with respect to the second movable body 50 toward the retraction direction enables a retraction movement of only the second movable body 50 in a state where the combination of the second movable body 50 with the first movable body 40 has been released. In other words, the retraction movement of the first movable body 40 is not made in a state where the combination of the first movable body 40 with the second movable body 50 has been released.

As described above, since the non-piercing end of the needle 30 is secured to the second movable body 50, upon the retraction movement of only the second movable body, it is possible to make a retraction movement of only the needle 30 secured to the second movable body 50. While on the other hand, the non-piercing end of the cannula 20 is merely pressed by the at least one of the first movable body 40 and the second movable body 50. Namely, the cannula 20 is not secured to the second movable body 50. Thus, upon the retraction movement of only the second movable body, a retraction movement of the cannula 20 not being secured to the second movable body 50 is not made.

It is preferable that the second coil spring 60B has the biasing force which enables the second movable body 50 to be contacted with the rear wall 19 of the casing main portion 13 in the retraction direction of the needle 30. Thus, it is possible to suitably make the retraction movement of the second movable body 50, which enables an entire needle 30 whose one of the end portions is secured to the second movable body 50 to be suitably moved in the retraction direction into the casing 10.

According to an embodiment of the present invention, it is preferable that the second coil spring 60B upon a release of its compression state is configured to be able to extend beyond at least one of end portions of the first coil spring 60A upon a release of its compression state. According to the configuration, an amount of the retraction movement of the second movable body 50 by the second coil spring 60B can be made relatively larger than an amount of the advance movement of the first movable body 40 by the first coil spring 60A. Thus, it is possible to suitably make the retraction movement of the needle 30 secured to the second movable body 50.

### (Holding member 90)

Furthermore, the cannula inserter 100 according to an embodiment of the present invention may further include the holding member 90. The holding member 90 is positioned in the internal space region 13a of the casing main portion 13 and is also positioned to be able to hold the cannula 20 upon the completion of its advance movement. As an example, the holding member 90 is positioned at the front region of the casing main portion 13.

As described above, the respective advance movements of the first movable body 40 and the second movable body 50 together can be made by the first coil spring 60A, and then the retraction movement of only the second movable body 50 can be made by the second coil spring 60B. Thus, the first movable body 40 in the state of the completion of the advance movement remains to be in the contact with the front wall 17 of the casing main portion 13. Thus, the cannula 20 in the state of the completion of the advance movement can remain to be positioned in the skin surface of the patient or the like after being launched from the casing 10 to the outside.

In this regard, a provision of the holding member 90 makes it possible to suitably hold the cannula 20 in the state of the completion of the advance movement in the holding member 90. The holding of the cannula 20 by the holding member 90 results from a mutual engagement of the cannula 20 in the state of the completion of the advance movement with the holding member 90, which makes it possible to suitably prevent the cannula 20 in the state of the completion of the advance movement from being moved toward the retraction direction. Thus, the cannula 20 can suitably remain to be positioned in the skin surface of the patient or the like.

The mutual engagement of the cannula 20 in the state of the completion of the advance movement with the holding member 90 can be achived by the following embodiments, for example.

As an example, as shown in Figs. 1J and 1K, the holding member 90 has a clearance through which the cannula 20 can pass to be launched to the outside. The clearance is formed between latches 91, 92 of the holding member 90, the latches 91, 92 being opposed to each other and also being capable of engaging with the non-piercing end side of the cannula 20. Furthermore, at least the non-piercing end side of the cannula 20 has a flexibility, and a hole-diameter W₁ of the non-piercing end side of the cannula 20 is larger than a hole-diameter W₂ of another portion other than the non-piercing end side of the cannula 20. A dimension W₃ of the clearance between the latches 91, 92 opposed to each other of the holding member 90 is smaller than the hole-diameter W₁ of the non-piercing end side of the cannula 20 at a point in time when the cannula 20 has passed through the clearance.

According to the above configuration, the following effects may be obtained even if a force toward the retraction direction acts after the launching of the cannula 20 through the holding member 90 to the outside.

Specifically, the non-piercing end side of the cannula 20 can be engaged with the latches 91, 92 of the holding member 90, since the hole-diameter W₁ of the non-piercing end side of the cannula 20 is larger than the hole-diameter W₂ of another portion other than the non-piercing end side of the cannula 20 and also the dimension W₃ of the clearance of the holding member 90 is smaller than the hole-diameter W₁ of the non-piercing end side of the cannula 20 at a point in time when the cannula 20 has passed through the clearance. Thus, the cannula 20 in the state of the completion of its advance movement can be engaged with the holding member 90, which makes it possible to preferably prevent the cannula 20 in the state of the completion of the advance movement from being moved toward the retraction direction. Thus, the cannula 20 can suitably remain to be positioned in the skin surface of the patient or the like.

### Motion of Cannula Inserter

A motion of the cannula inserter 100 according to an embodiment of the present invention will be described hereinafter.

### Motion of Cannula Inserter in pre-motion state

A motion of the cannula inserter 100 in a pre-motion state will be described hereinafter (see Figs. 1A to 1D). Descriptions overlapping with the descriptions in the section of the configuration of the cannula inserter will be simplified or omitted.

In a pre-motion state, the engaging portion 50A of the second movable body 50 and the protrusion portion 40D of the first movable body 40 are configured to be able to be engaged with each other. Thus, the first movable body 40 and the second movable body 50 are configured together with each other. Namely, the first movable body 40 and the second movable body are combined together with each other. Furthermore, as described above, the at least one of the first movable body 40 and the second movable body 50 combined together with each other is held by the trigger member 70 in the pre-motion state.

As described above, each of the first coil spring 60A and the second coil spring 60B is in the compression state before the use thereof. The first coil spring 60A is arranged between the casing 10 and the first movable body 40 such that the one of the end portions of the first coil spring 60A is secured to the casing 10 and the other of the end portions of the first coil spring 60A is secured to the first movable body 40. The second coil spring 60B is arranged between the first movable body 40 and the second movable body 50 such that the one of the end portions of the second coil spring 60B is secured to the first movable body 40 and the other of the end portions thereof is secured to the second movable body 50.

According to the above configuration, the first movable body 40 and the second movable body 50 combined together can be in a standby state in the casing main portion 13, specifically at the rear wall 19-side of the casing main portion 13, the combined body of the first movable body 40 and the second movable body 50 having the first coil spring 60A and the second coil spring 60B each of which is in the compression state therein.

Thus, the needle 30 itself secured to the second movable body 50 can be in the standby state in the casing main portion 13. Furthermore, the first movable body 40 and the second movable body 50 are both in the standby state in the casing main portion 13 without the advance movement of them. Thus, the cannula 20 is not pressed by the at least one of the first movable body 40 and the second movable body 50.

Thus, the cannula 20 can also be in a standby state in the casing main portion 13. This enables the cannula 20 and the needle 30 to be prevented from being launched from the inside of the casing 10 to the outside (e.g., the skin surface of the patient or the like), which enables respective piercing ends of the cannula 20 and the needle 30 not to be exposed. Specifically, it is possible to prevent the cannula 20 and the needle 30 from being launched from the opening of the insulin administration device having the cannula inserter 100 therein to the outside, which enables the respective piercing ends of the cannula 20 and the needle 30 not to be exposed.

### Motion of Cannula Inserter in first motion state corresponding to advance state of needle and cannula

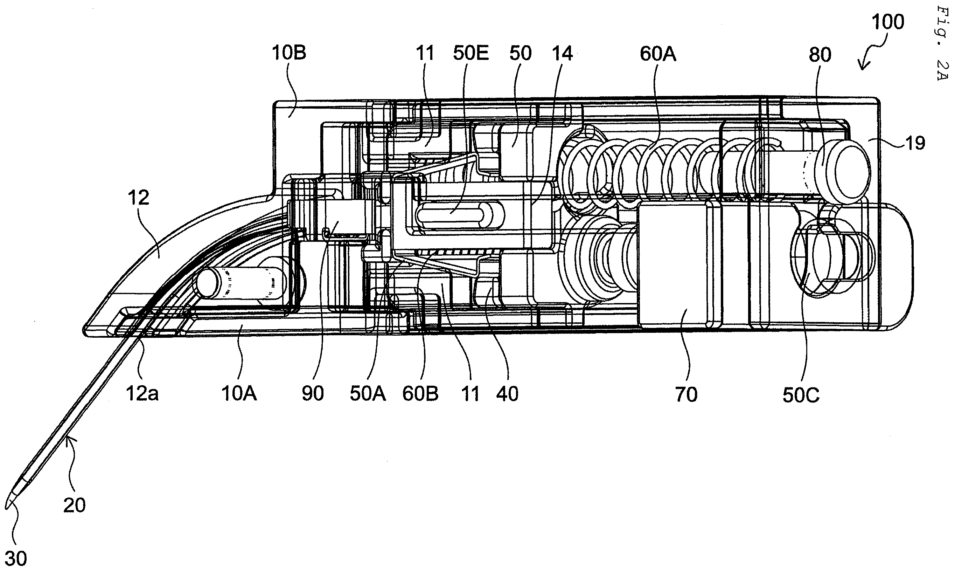

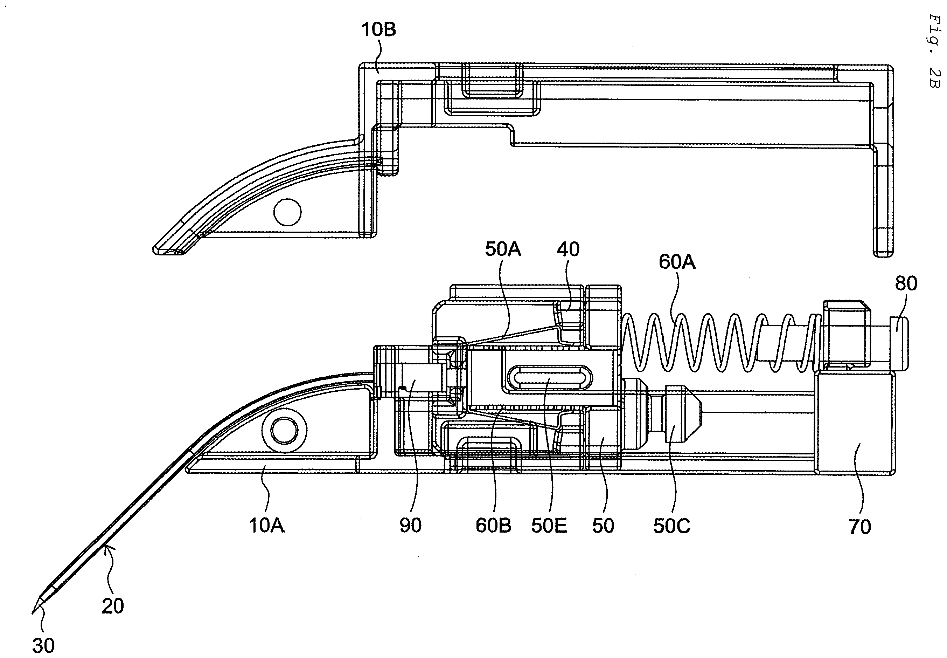

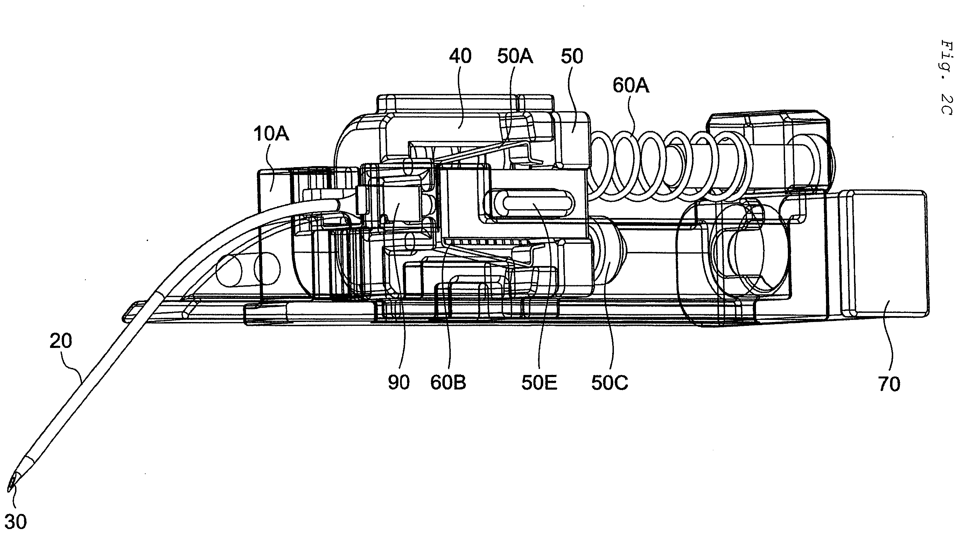

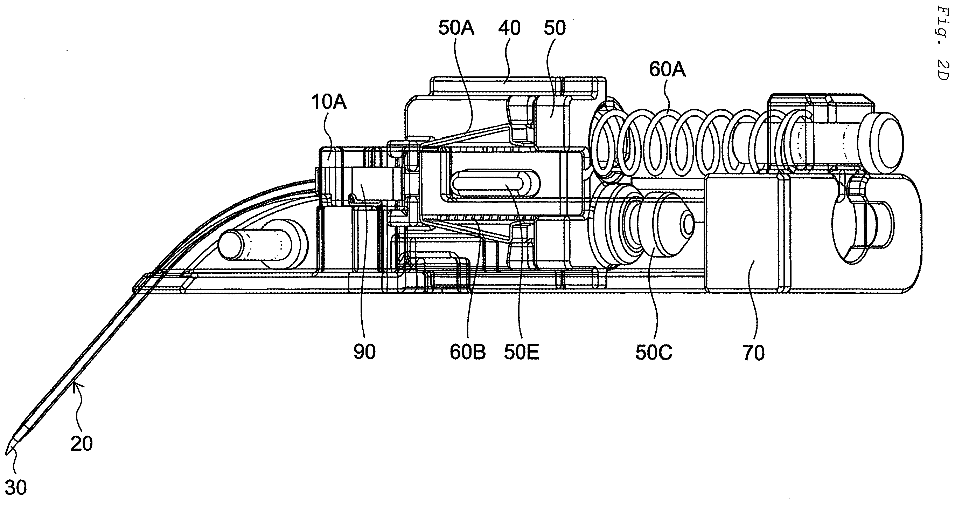

A motion of the cannula inserter 100 in a first motion state will be described hereinafter (see Figs. 2A to 2D).

In the first motion state, the trigger member 70 is driven firstly. A mechanism for driving the trigger member 70 may be a manual type or an electrically controlled automatic type. According to an embodiment shown in Figures, the trigger member 70 is pushed, which enables a release of a holding state of the trigger member 70 with the at least one of the first movable body 40 and the second movable body 50 combined together with each other. As described above, the trigger member 70 is configured to be able to hold the extension portion 50C of the second movable body 50 as an example.

When its holding state is released, the holding relationship between the second movable body 50 and the trigger member 70 is disengaged. Thus, the disengagement of the holding relationship between the trigger member 70 and the combined body of "the first movable body 40 combined together with the second movable body 50" and "the second movable body 50". As described above, each of the first coil spring 60A and the second coil spring 60B is in the compression state before the use thereof. The first coil spring 60A in the compression state is capable of extending in the advance direction of the needle 30 to bias the first movable body 40. The first coil spring 60A is arranged between the casing 10 and the first movable body 40 such that the one of the end portions thereof is secured to the casing 10 and the other of the end portions thereof is secured to the first movable body 40.

According to the above configuration, the disengagement of the holding relationship causes an action of the biasing force in the advance direction of the first coil spring 60A to the first movable body 40, which makes it possible to make the advance movement of both of the first movable body 40 biased by the first coil spring 60A and the second movable body 50 combined with the first movable body 40. Thus, the first movable body 40 and the second movable body 50 combined together can be changed from a non-motion state to a motionable state.

As described above, the non-piercing end of the needle 30 is secured to the second movable body 50, and the non-piercing end of the cannula 20 is configured to be able to be pressed by the at least one of the first movable body 40 and the second movable body 50. Thus, the advance movement of both of the first movable body 40 and the second movable body 50 enables an advance movement of the needle 30 secured to the second movable body 50 as well as the cannula 20 configured to be pressed by the movable body.

Accordingly, the cannula 20 and the needle 30 can be launched from the inside of the casing 10 to the outside (e.g., the skin surface of the patient or the like), and thus respective piercing ends of the cannula 20 and the needle 30 can be exposed. Specifically, it is possible to launch the cannula 20 and the needle 30 from the opening of the insulin administration device having the cannula inserter 100 therein to the outside, and thus the respective piercing ends of the cannula 20 and the needle 30 can be exposed.

The first motion state maintains the engagement of "the engaging portion 50A of the second movable body 50 with the protrusion 40D of the first movable body 40" for the combination of the first movable body 40 with the second movable body 50. The combination of the first movable body 40 with the second movable body 50 remains to be maintained due to the maintenance of the engagement of the engaging portion 50A of the second movable body 50 with the protrusion 40D of the first movable body 40.

As described above, each of the first coil spring 60A and the second coil spring 60B is in the compression state before the use thereof, and also the second coil spring 60B in the compression state is capable of extending in the retraction direction of the needle 30 to bias the second movable body 50. The second coil spring 60B is arranged between the first movable body 40 and the second movable body 50 such that the one of the end portions thereof is secured to the first movable body 40 and the other of the end portions thereof is secured to the second movable body 50.

According to the above arrangement, in the state of the maintenance of the combination of the first movable body 40 with the second movable body 50, the second coil spring 60B remains to be in the compression state without being subjected to be in a use state. Thus, there is not a change in a distance between the first movable body 40 and the second movable body 50 in an interconnection with each other via the second coil spring 60B upon the advance movement of the two movable bodies which remain to be combined together with each other.

### Motion of Cannula Inserter in second motion state corresponding to retraction state of needle and advance state of cannula

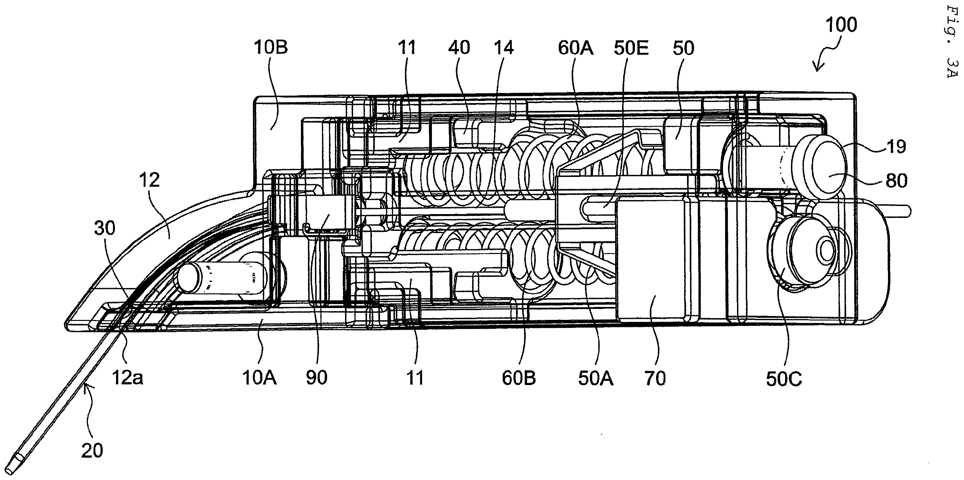

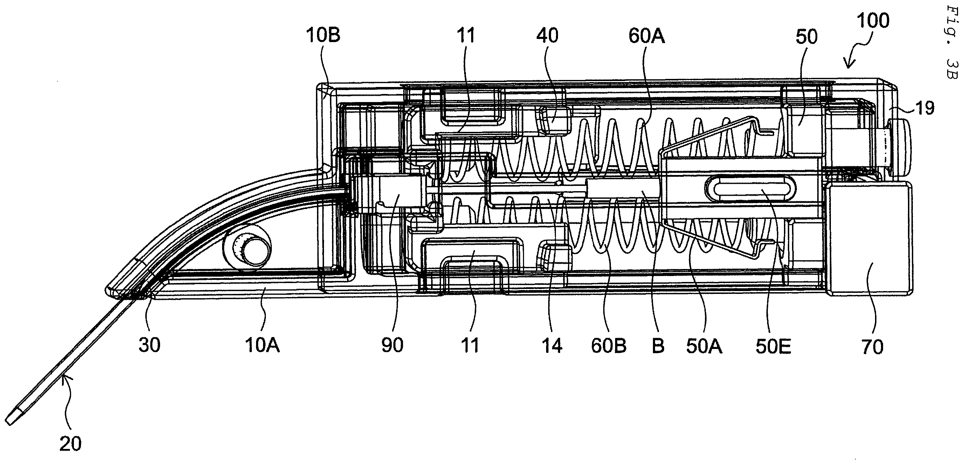

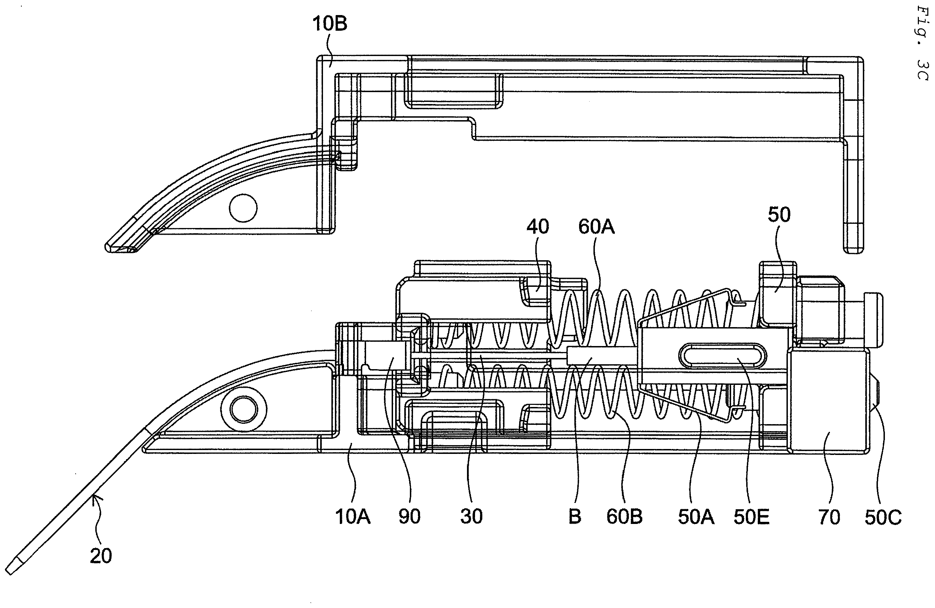

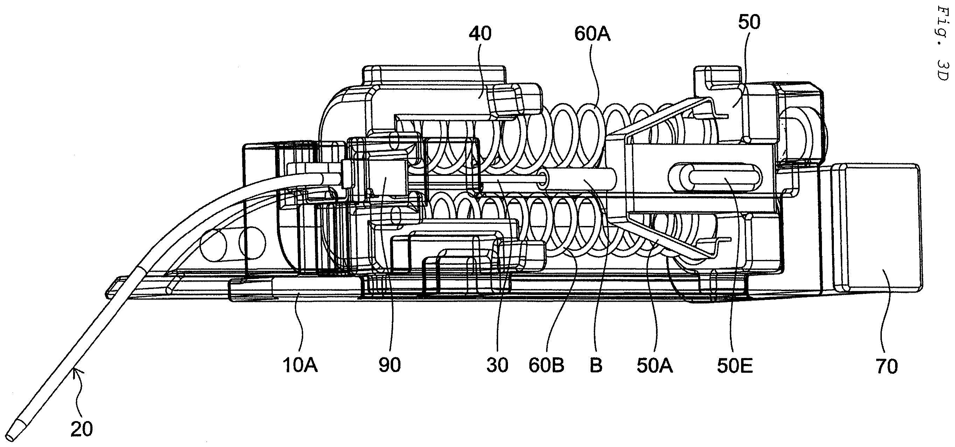

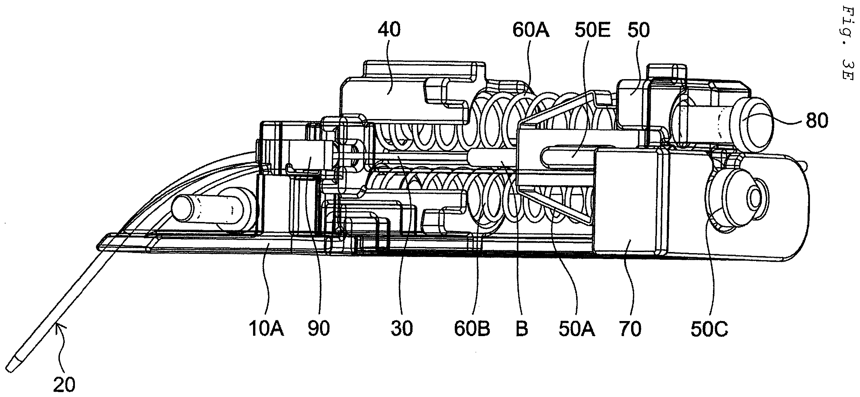

A motion of the cannula inserter 100 in a second motion state will be described hereinafter (see Figs. 3A to 3E).

As described above, in the first motion state, the first movable body 40 biased by the first coil spring 60A and the second movable body 50 combined with the first movable body 40 can be moved together in the advance direction. Upon the advance movement of the two movable bodies 40, 50 toward the front wall 17 of the casing main portion 13, the first movable body 40 is in a contact with the convex region 17a of the front wall 17, which enables the advance movement of the combined body of the first movable body 40 and the second movable body 50 to be completed.

According to an embodiment of the present invention, the convex portion 11 on the inner surface of the casing main portion 13 is positioned to be able to be in the contact with the flexible engaging portion 50A of the second movable body 50 upon the completion of the movement in the advance direction. Thus, upon the completion of the advance movement of the combined body of the first movable body 40 and the second movable body 50, the flexible engaging portion 50A of the second movable body 50 can be in the contact with the convex portion 11 on the inner surface of the casing main portion 13.

The convex portion 11A on the side of the first casing cover member 10A and the convex portion 11B on the side of the second casing cover member 10B may be used as respective convex portions 11. The convex portion 11A may be formed at the front region of the portion 13A of the casing main portion 13 and may be formed on the inner surface of the lower wall 16A, for example. The convex portion 11B may be formed at the front region of the portion 13B of the casing main portion 13 and may be formed on the inner surface of the upper wall 15B, for example.

Due to the convex portions 11A, 11B, upon the completion of the advance movement of the second movable body 50, the engaging portions 50A of the second movable body 50 can be in the contact with the convex portions 11A, 11B, respectively, thereby enabling the flexible engaging portions 50A to be flexed. Thus, each engaging portion 50A can be spaced apart from the protrusion portion 40D of the first movable body 40, and thus the engagement of the first movable body 40 with the second movable body 50 can be released. As a result, the combination of the first movable body 40 with the second movable body 50 can be released.

As described above, the second coil spring 60B is arranged between the first movable body 40 and the second movable body 50 such that the one of end portions thereof is secured to the first movable body 40 and the other of the end portions thereof is secured to the second movable body 50. The second coil spring 60B is in the compression state before the use thereof, and the second coil spring 60B in the compression state is capable of extending in the retraction direction of the needle 30 to bias the second movable body 50. According to the above arrangement, the release of the combination of the first movable body 40 with the second movable body 50 causes a release of the compression state of the second coil spring 60B whose one end portion is secured to the first movable body 40 and whose other end portion is secured to the second movable body 50.

Thus, upon the compression release of the second coil spring 60B, the second coil spring 60B in the compression state can extend from the one end side thereof secured to the first movable body 40 toward the other end side thereof secured to the second movable body 50, which enables the second movable body 50 to be biased in the retraction direction. Thus, it is possible to make the retraction movement of only the second movable body 50 in the state where the combination of the second movable body 50 with the first movable body 40 has been released. In other words, the retraction movement of the first movable body 40 is not made in the state where the combination of the first movable body 40 with the second movable body 50 has been released.

Furthermore, since the non-piercing end of the needle 30 is secured to the second movable body 50, upon the retraction movement of only the second movable body, it is possible to make the retraction movement of only the needle 30 secured to the second movable body 50. While on the other hand, the non-piercing end of the cannula 20 is merely pressed by the at least one of the first movable body 40 and the second movable body 50. Namely, the cannula 20 is not secured to the second movable body 50. Thus, upon the retraction movement of only the second movable body, the retraction movement of the cannula 20 not being secured to the second movable body 50 is not made.

Upon the release of the combination of the first movable body 40 with the second movable body 50, the first movable body 40 remains to maintain the biased state by the first coil spring 60A. Thus, the first movable body 40 upon the completion of its advance movement can remain to maintain its contact state with the convex region 17a of the front wall 17 of the casing main portion 13.

Accordingly, it is possible to shift only the needle 30 from its launch state to the outside to its retraction state, while remaining to be the state where the cannula 20 is launched from the inside of the casing 10 to the outside (e.g., the skin surface of the patient or the like). Specifically, the retraction movement of only the needle 30 can be made such that the piercing end of the needle 30 is positioned in the passage of the casing protrusion portion 12.

As described above, according to an embodiment of the present invention, the holding member 90 is positioned to be able to hold the cannula 20 upon the completion of its advance movement. Thus, it is possible to suitably hold the cannula 20 in the state of the completion of the advance movement by the holding member 90. This results from a mutual engagement of the cannula 20 in the state of the completion of the advance movement with the holding member 90. Thus, the cannula 20 upon completion of the advance movement can be suitably prevented from being moved toward the retraction direction. Accordingly, the cannula 20 can suitably remain to be positioned in the skin surface of the patient or the like.

Although some embodiments of the present invention have been hereinbefore described, these are merely typical examples in the present invention. It will be readily appreciated by the skilled person that various modifications are possible without departing from the scope of the present invention.

It should be noted that the present invention as described above includes the following aspects.
The first aspect: A cannula inserter, comprising
   a cannula, a needle, a first movable body, a second movable body, and a biasing means comprising a first coil spring and a second coil spring in a casing, the needle being able to be moved in the cannula,
   wherein the first coil spring and the second coil spring each of which is in a compression state before a use are arranged side by side in parallel, the first coil spring is capable of extending in an advance direction of the needle to bias the first movable body, and the second coil spring is capable of extending in a retraction direction of the needle to bias the second movable body,
   wherein a non-piercing end of the needle is secured to the second movable body, and a non-piercing end of the cannula is configured to be pressed by at least one of the first movable body and the second movable body,
   wherein the compression state of the first coil spring is released such that the first movable body is biased by the first coil spring, thereby enabling the first movable body and the second movable body which are combined with each other to be moved in the advance direction of the needle,
   wherein, after a completion of a movement of the first movable body and the second movable body to a predetermined position in the advance direction, a combination of the first movable body with the second movable body is released such that the compression state of the second coil spring is configured to be able to be released, and
   wherein the second movable body is biased by the second coil spring upon a release of its compression state, thereby enabling the second movable body to be moved in the retraction direction of the needle.
The second aspect: The cannula inserter according to the first aspect, wherein the second coil spring upon the release of its compression state is capable of extending beyond at least one of end portions of the first coil spring upon that of its compression state.
The third aspect: The cannula inserter according to the first aspect or the second aspect, wherein a combined movement of the first movable body with the second movable body in the advance direction enables an advance movement of both of the needle and the cannula.
The fourth aspect: The cannula inserter according to any one of the first to third aspects, wherein a movement of the second movable body in the retraction direction enables a retraction movement of the needle secured to the second movable body. The fifth aspect: The cannula inserter according to any one of the first to fourth aspects,
The sixth aspect: The cannula inserter according to any one of the first to fifth aspects, wherein the first movable body and the second movable body are configured to be mutually engaged with each other to be able to be combined with each other.
The seventh aspect: The cannula inserter according to the sixth aspect, wherein the second movable body comprises an engaging portion having a flexibility, the first movable body comprises a protrusion as an engaged portion, and an engagement of the engaging portion of the second movable body with respect to the protrusion of the first movable body enables the first movable body and the second movable body to be engaged with each other. The eighth aspect: The cannula inserter according to the seventh aspect, wherein the engaging portion of the second movable body is a flexible wing portion.
The ninth aspect: The cannula inserter according to any one of the sixth to eighth aspects, wherein a release of the engagement of the first movable body with the second movable body causes a combination of the first movable body with the second movable body to be released.
The tenth aspect: The cannula inserter according to any one of the first to ninth aspects, wherein the casing comprises a convex portion on an inner surface and
   wherein the convex portion is positioned such that the engaging portion of the second movable body upon a completion of a movement thereof in the advance direction can be in a contact with the convex portion.
The eleventh aspect: The cannula inserter according to the tenth aspect appended to the seventh aspect, wherein the contact of the engaging portion of the second movable body with the convex portion of the casing enables a release of the engagement of the engaging portion with the protrusion of the first movable body.
The twelfth aspect: The cannula inserter according to any one of the first to eleventh aspects, wherein the first coil spring is arranged between the casing and the first movable body such that one of end portions of the first coil spring is secured to the casing and other of the end portions thereof is secured to the first movable body.
The thirteenth aspect: The cannula inserter according to any one of the first to twelfth aspects, wherein the second coil spring is arranged between the first movable body and the second movable body such that one of end portions of the second coil spring end is secured to the first movable body and other of the end portions end thereof is secured to the second movable body.
The fourteenth aspect: The cannula inserter according to any one of the first to thirteenth aspects, wherein the first coil spring and the second coil spring have the substantially same diameter as each other.
The fifteenth aspect: The cannula inserter according to any one of the first to fourteenth aspects, wherein a holding member is arranged on an inner surface of the casing, the holding member being capable of holding the cannula upon a completion of a movement thereof in the advance direction.
The sixteenth aspect: The cannula inserter according to the fifteenth aspect, wherein the holding member is configured to be able to engage with the cannula upon the completion of the movement thereof to hold the cannula.
The seventeenth aspect: The cannula inserter according to any one of the first to sixteenth aspects, wherein at least a non-piercing end side of the cannula has a flexibility, and the non-piercing end side of the cannula has a hole-diameter larger than that of another portion other than the non-piercing end side of the cannula.
The eighteenth aspect: The cannula inserter according to the seventeenth aspect appended to the fifteenth aspect, wherein the holding member has a clearance through which the cannula can pass, and the clearance of the holding member has a size smaller than the hole-diameter of the non-piercing end side of the cannula.
The nineteenth aspect: The cannula inserter according to any one of the first to eighteenth aspects, wherein at least one of the first movable body and the second movable body before its movement in the advance direction is held by a trigger member.
The twentieth aspect: An insulin administration device comprising the cannula inserter according to any one of the first to nineteenth aspects.
The twenty first aspect: The insulin administration device according to the twentieth aspect, comprising an opening through which the cannula and the needle can pass, and wherein the opening is sealed before launching the cannula and the needle to an outside of the insulin administration device.

### INDUSTRIAL APPLICABILITY

The cannula inserter according to an embodiment of the present invention can be used to regularly administer a small amount of insulin.

### CROSS REFERENCE TO RELATED PATENT APPLICATION

The present application claims the right of priority of Japanese Patent Application No. 2019-014567 (filed on January 30, 2019, the title of the invention: "CANNULA INSERTER"), the disclosure of which is incorporated herein by reference.

### EXPLANATION OF REFERENCE NUMERALS

100 Cannula inserter
10 Casing
10A First casing cover member
10B Second casing cover member
11 Convex portion on inner surface of casing
11A Convex portion on inner surface of casing
11B Convex portion on inner surface of casing
12 Casing protrusion portion
12a Opening for launch
12A A portion of casing protruding portion
12B A portion of casing protruding portion
13 Casing main portion
13a Internal space region of casing main portion
13A A portion of casing main portion
14, 14B Clearance on surface of casing main portion
15 Upper wall of casing main portion
16 Lower wall of casing main portion
16A Lower wall of a portion of casing main portion
17 Front wall of casing main portion
17A Front wall of a portion of casing main portion
18 Side wall of casing main portion
18A Side wall of a portion of casing main portion
19 Rear wall of casing main portion
19a Opening in Rear wall of casing main portion
19b Opening in Rear wall of casing main portion
19A Rear wall of a portion of casing main portion
13B A portion of casing main portion
15B Upper wall of a portion of casing main portion
17B Front wall of a portion of casing main portion 18B Side wall of a portion of casing main portion
19B Rear wall of a portion of casing main portion
20 Cannula
30 Needle
40 First movable body
40A Support portion of first movable body
41A Support region for first coil spring
41B Support region for second coil spring
40B Front wall portion of first movable body
40C Side wall portion of first movable body
40D Protrusion portion of first movable body
40E Protrusion on inner side surface of front wall portion of first movable body
40F Protrusion on inner side surface of front wall portion of first movable body
40G Convex portion on surface of first movable body
50 Second movable body
50A Flexible engaging portion capable of being engaged with protrusion portion of first movable body
50B Body portion of second movable body
50C Extension portion of second movable body
50D Front wall of body portion of second movable body
50E Convex portion on surface of second movable body
50F Protrusion 50F on body portion of second movable body
50G Rear wall of body portion of second movable body
51A Main surface of engaging portion of second movable body
52A Side surface of engaging portion of second movable body
53A Wing of engaging portion of second movable body
54A Latch at end portion of wing of engaging portion of second movable body
60 Biasing means
60A First coil spring
60B Second coil spring
70 Trigger member
80 Shaft member
90 Holding member
91 Latch of holding member
92 Latch of holding member
B Intercalated member
W₁ Hole-diameter of non-piercing end side of cannula
W₂ Hole-diameter of another portion other than non-piercing end side of cannula
W₃ Dimension of clearance of holding member

## Claims

1. A cannula inserter, comprising
a cannula, a needle, a first movable body, a second movable body, and a biasing means comprising a first coil spring and a second coil spring in a casing, the needle being able to be moved in the cannula,
wherein the first coil spring and the second coil spring each of which is in a compression state before a use are arranged side by side in parallel, the first coil spring is capable of extending in an advance direction of the needle to bias the first movable body, and the second coil spring is capable of extending in a retraction direction of the needle to bias the second movable body,
wherein a non-piercing end of the needle is secured to the second movable body, and a non-piercing end of the cannula is configured to be pressed by at least one of the first movable body and the second movable body,
wherein the compression state of the first coil spring is released such that the first movable body is biased by the first coil spring, thereby enabling the first movable body and the second movable body which are combined with each other to be moved in the advance direction of the needle,
wherein, after a completion of a movement of the first movable body and the second movable body to a predetermined position in the advance direction, a combination of the first movable body with the second movable body is released such that the compression state of the second coil spring is configured to be able to be released, and
wherein the second movable body is biased by the second coil spring upon a release of its compression state, thereby enabling the second movable body to be moved in the retraction direction of the needle.

2. The cannula inserter according to claim 1, wherein the second coil spring upon the release of its compression state is capable of extending beyond at least one of end portions of the first coil spring upon that of its compression state.

3. The cannula inserter according to claim 1 or 2, wherein a combined movement of the first movable body with the second movable body in the advance direction enables an advance movement of both of the needle and the cannula.

4. The cannula inserter according to any one of claims 1 to 3, wherein a movement of the second movable body in the retraction direction enables a retraction movement of the needle secured to the second movable body.

5. The cannula inserter according to any one of claims
1 to 4, wherein the compression state of the second coil spring is configured to be able to be released after a completion of a release of the compression state of the first coil spring.

6. The cannula inserter according to any one of claims 1 to 5, wherein the first movable body and the second movable body are configured to be mutually engaged with each other to be able to be combined with each other.

7. The cannula inserter according to claim 6, wherein the second movable body comprises an engaging portion having a flexibility, the first movable body comprises a protrusion as an engaged portion, and an engagement of the engaging portion of the second movable body with respect to the protrusion of the first movable body enables the first movable body and the second movable body to be engaged with each other.

8. The cannula inserter according to claim 7, wherein the engaging portion of the second movable body is a flexible wing portion.

9. The cannula inserter according to any one of claims 6 to 8, wherein a release of the engagement of the first movable body with the second movable body causes a combination of the first movable body with the second movable body to be released.

10. The cannula inserter according to any one of claims 1 to 9, wherein the casing comprises a convex portion on an inner surface and
wherein the convex portion is positioned such that the engaging portion of the second movable body upon a completion of a movement thereof in the advance direction can be in a contact with the convex portion.

11. The cannula inserter according to claim 10 appended to claim 7, wherein the contact of the engaging portion of the second movable body with the convex portion of the casing enables a release of the engagement of the engaging portion with the protrusion of the first movable body.

12. The cannula inserter according to any one of claims 1 to 11, wherein the first coil spring is arranged between the casing and the first movable body such that one of end portions of the first coil spring is secured to the casing and other of the end portions thereof is secured to the first movable body.

13. The cannula inserter according to any one of claims 1 to 12, wherein the second coil spring is arranged between the first movable body and the second movable body such that one of end portions of the second coil spring end is secured to the first movable body and other of the end portions end thereof is secured to the second movable body.

14. The cannula inserter according to any one of claims 1 to 13, wherein the first coil spring and the second coil spring have the substantially same diameter as each other.

15. The cannula inserter according to any one of claims 1 to 14, wherein a holding member is arranged on an inner surface of the casing, the holding member being capable of holding the cannula upon a completion of a movement thereof in the advance direction.

16. The cannula inserter according to claim 15, wherein the holding member is configured to be able to engage with the cannula upon the completion of the movement thereof to hold the cannula.

17. The cannula inserter according to any one of claims 1 to 16, wherein at least a non-piercing end side of the cannula has a flexibility, and the non-piercing end side of the cannula has a hole-diameter larger than that of another portion other than the non-piercing end side of the cannula.

18. The cannula inserter according to claim 17 appended to claim 15, wherein the holding member has a clearance through which the cannula can pass, and the clearance of the holding member has a size smaller than the hole-diameter of the non-piercing end side of the cannula.

19. The cannula inserter according to any one of claims 1 to 18, wherein at least one of the first movable body and the second movable body before its movement in the advance direction is held by a trigger member.

20. An insulin administration device comprising the cannula inserter according to any one of claims 1 to 19.

21. The insulin administration device according to claim 20, comprising an opening through which the cannula and the needle can pass, and wherein the opening is sealed before launching the cannula and the needle to an outside of the insulin administration device.
released such that the compression state of the second coil spring is configured to be able to be released, and wherein the second movable body is biased by the second coil spring upon a release of its compression state, thereby enabling the second movable body to be moved in the retraction direction of the needle.
